# EUROPEAN PATENT SPECIFICATION

(11) **EP 1 901 994 B1**
(45) Date of publication and mention of the grant of the patent: **27.01.2010**
(21) Application number: 06754663.0
(22) Date of filing: 04.07.2006
(51) Int. Cl.: C01B 21/14, C07C 249/04, C07C 239/00

(54) **PROCESS FOR THE CONTINUOUS PRODUCTION OF HYDROXYLAMMONIUM**
VERFAHREN ZUR KONTINUIERLICHEN PRODUKTION VON HYDROXYLAMMONIUM
PROCEDE DE FABRICATION EN CONTINU D'HYDROXYLE AMMONIUM

(30) Priority: 08.07.2005 WO PCT/NL2005/000491
(43) Date of publication of application: 26.03.2008
(73) Proprietor: DSM IP Assets B.V., 6411 TE Heerlen (NL)
(72) Inventor: OEVERING, Hendrik, NL-6181 BR Elsloo (NL); POORTER, Olaf, NL-6171 WS Stein (NL)
(74) Representative: Hoogendam, Gerrie Christine
(86) International application number: PCT/EP2006/006507
(87) International publication number: WO 2007/006458

(56) References cited:
- EP-A1- 0 059 366
- US-A- 3 940 442
- "METHOD FOR PROLONGING SERVICE LIFE OF HYDROXYLAMINE CATALYST" EPODOC, 22 January 2003 (2003-01-22), XP002354067 & CN 1 391 981 A (BALING BRANCH CO CHINA PETROCH [CN]) 22 January 2003 (2003-01-22) cited in the application

## Description

The invention relates to a process for the continuous production of hydroxyl ammonium in a hydroxyl ammonium synthesis zone by reduction of nitrate ions or nitrogen oxides with hydrogen in the presence of a catalyst. The invention further relates to a process for the continuous production of cyclohexanone oxime in which an acid-buffered inorganic process liquid is continuously cycled from a hydroxyl ammonium synthesis zone to a cyclohexanone oxime synthesis zone and back to the hydroxyl ammonium synthesis zone, in which hydroxyl ammonium synthesis zone hydroxyl ammonium is formed by reduction of nitrate ions or nitrogen oxides with hydrogen in the presence of a catalyst, and in which cyclohexanone oxime synthesis zone hydroxyl ammonium is reacted with cyclohexanone to form cyclohexanone oxime. In addition the invention relates to an equipment for the continuous production of cyclohexanone oxime in a cyclohexanone oxime synthesis zone which is connected to a hydroxyl ammonium synthesis zone in such a way that an acid-buffered inorganic process liquid can be cycled continuously from the hydroxyl ammonium synthesis zone to the cyclohexanone oxime synthesis zone and back to the hydroxyl ammonium synthesis zone.

The inorganic process liquid is acid-buffered by means of for instance phosphoric acid and/or hydrogen sulphate and the buffer salts derived from these acids. In the hydroxyl ammonium synthesis zone nitrate ions or nitrogen oxides are converted with hydrogen to hydroxylamine. The hydroxylamine reacts with the free buffer acid to form the corresponding hydroxyl ammonium, which is then isolated or transported as a salt e.g. to a cyclohexanone oxime synthesis zone. In a cyclohexanone oxime synthesis zone the hydroxyl ammonium salt reacts with a ketone to form the corresponding oxime, with release of acid. After separation of the oxime from the inorganic process liquid, the inorganic process liquid may be returned to the hydroxyl ammonium synthesis zone, whereby fresh nitrate ions or nitrogen oxides are being supplied to the inorganic process liquid. If the hydroxyl ammonium synthesis starts from a solution of phosphoric acid and nitrate, the net chemical reactions occurring during the process can be represented by the following equations:
1) Preparation of the hydroxyl ammonium:

   2 H₃PO₄ + NO₃⁻ + 3 H₂ → NH₃OH⁺ + 2 H₂PO₄ ⁺ 2 H₂O
2) Preparation of the oxime 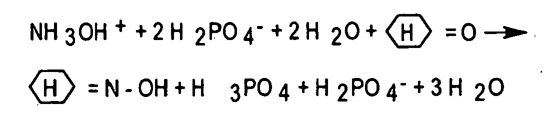
3) Supply of HNO₃ to make up the depletion of the source of nitrate ions after removal of the oxime formed

   H₂PO⁻₄ + HNO₃ → H₃PO₄ + NO⁻₃

The hydroxyl ammonium preparation according to equation 1 is catalyzed heterogeneously. The catalyst applied in this preparation mostly consists of a metal from the platinum metal group, for instance Pd or Pd+Pt, as active component on a carrier material such as carbon. It is known that with a Pd+Pt-on-carbon catalyst a high activity and a reasonable selectivity regarding the reaction of nitrate ions or nitrogen oxides to hydroxyl ammonium salt is achieved. With a Pd-on-carbon catalyst a higher selectivity concerning the hydroxyl ammonium salt synthesis is achieved. But under comparable conditions the activity achieved with this catalyst is lower, so that mostly a Pd+Pt-on-carbon catalyst is preferred. However, in use such catalysts deactivate and lose selectivity, which requires addition and/or substitution of the catalyst after being used for a certain period of time.

A method for prolonging the working life of such a catalyst is disclosed in CN-1391981. In the method of CN-1391981 3-15% of deactivated catalyst is removed and 3-15% of activated catalyst is added when a fall in selectivity and activity of the total amount of catalyst has reached 1.7-1.8 times that of the total amount of the original catalyst, maintaining the total amount of catalyst without increase. Although such a method is helpful in performing a process with an acceptable activity and selectivity for a prolonged period of time at a certain moment it still is necessary to replace all the catalyst and consequently to discontinue the whole process. The drawback of discontinuing the whole process is a serious drop in production due to discontinuing and restarting the process.

The object of the present invention is to provide an improved method for the replacement of deactivated catalyst in a process for the continuous production of hydroxyl ammonium formed by reduction of nitrate ions or nitrogen oxides with hydrogen in the presence of a catalyst.

This object is achieved by providing a process whereby hydroxyl ammonium is produced in the hydroxyl ammonium synthesis zone in 2 or more parallel placed hydroxyl ammonium production units.

The advantage of the process according to the present invention is that the hydroxyl ammonium production process can be continued while a part of the deactivated catalyst is replaced by fresh highly active and selective catalyst. This replacement is performed in one of the parallel placed hydroxyl ammonium production units while the hydroxyl ammonium production process can be continued in 1 or more of the parallel placed hydroxyl ammonium production units. Such a process is advantageous above the process as e.g. disclosed in CN-1391981 whereby 3-15% of deactivated catalyst is removed and 3-15% of activated catalyst is added when a fall in selectivity and activity of the total amount of catalyst has reached 1.7-1.8 times that of the total amount of the original catalyst, maintaining the total amount of catalyst without increase, for several reasons as given below. In the process according to the present invention only deactivated catalyst of the same age is removed whereas in a process wherein 3-15% of deactivated catalyst is removed and 3-15% of activated catalyst is added only at the first change deactivated catalyst of the same age is removed, in all next changes catalyst having a different age is being removed. Accordingly, in the process according to the present invention the amount of hydroxyl ammonium produced per gram catalyst is at least approximately 10% higher than a process wherein catalyst is removed and added in one reactor. Another advantage of the process according to the present invention is that the production rate of hydroxyl ammonium is increased with approximately 10%. Still another advantage of the process according to the present invention above a process wherein part of the total amount of catalyst is replaced with fresh catalyst is the stability of the hydroxyl ammonium production process. In the process according to the present invention the catalyst is replaced in one production unit whereby the hydroxyl ammonium production process in the other production units is continued, whereas in a process in one production unit wherein part of the total amount of catalyst is replaced with fresh catalyst, once the process has to be discontinued completely which subsequently will require some time to re-establish a stable production process.

Moreover, the process according to the present invention is favorable for a highly flexible way of continuously producing hydroxyl ammonium. A stop of one of the parallel placed hydroxyl ammonium production units can also be used to clean that unit or perform other required operations without discontinuing the production of hydroxyl ammonium in the hydroxyl ammonium synthesis zone. It is also possible to discontinue the production of hydroxyl ammonium in one of the parallel placed hydroxyl ammonium production units in case the raw materials are scarce or in case that the consumption of hydroxyl ammonium in a follow-up process has declined. In addition to decreasing the production capacity by eliminating a production unit it is also simple to increase the production capacity by adding an additional production unit, which e.g. might be favorable for debottlenecking a follow-up process. An example of such a follow-up process is the production of cyclohexanone oxime. In a process for the continuous production of cyclohexanone oxime the process for continuously producing hydroxyl ammonium according to the present invention has a further advantage in that the necessity of applying a buffer stock of hydroxyl ammonium between the hydroxyl ammonium synthesis zone and the cyclohexanone oxime synthesis zone is reduced or even eliminated, since one of the parallel placed hydroxyl ammonium production units can be discontinued without discontinuing the continuous production of hydroxyl ammonium. Therefore the present invention is also related to a process for the continuous production of cyclohexanone oxime in which an inorganic process liquid is continuously cycled from a hydroxyl ammonium synthesis zone to a cyclohexanone oxime synthesis zone and back to the hydroxyl ammonium synthesis zone, in which hydroxyl ammonium synthesis zone hydroxyl ammonium is formed by reduction of nitrate ions or nitrogen oxides with hydrogen in the presence of a catalyst, and in which hydroxyl ammonium synthesis zone hydroxyl ammonium is reacted with cyclohexanone to form cyclohexanone oxime. Further the present invention is related to a process for decreasing or increasing the production capacity of a process for the continuous production of cyclohexanone oxime in which an inorganic process liquid is continuously cycled from a hydroxyl ammonium synthesis zone to a cyclohexanone oxime synthesis zone and back to the hydroxyl ammonium synthesis zone, in which hydroxyl ammonium synthesis zone hydroxyl ammonium is formed by reduction of nitrate ions or nitrogen oxides with hydrogen in the presence of a catalyst, and in which hydroxyl ammonium synthesis zone hydroxyl ammonium is reacted with cyclohexanone to form cyclohexanone oxime.

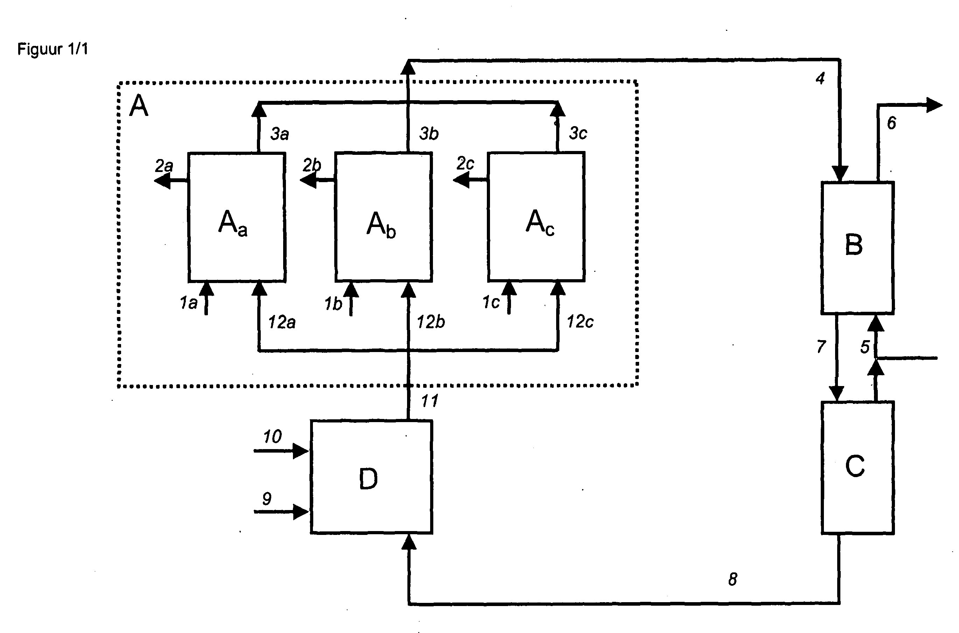

The present invention is still further related to an equipment for the continuous production of cyclohexanone oxime in a cyclohexanone oxime synthesis zone which is connected to a hydroxyl ammonium synthesis zone comprising 2 or more parallel placed hydroxyl ammonium production units, in such a way that an inorganic process liquid can be cycled continuously from the hydroxyl ammonium synthesis zone to the cyclohexanone oxime synthesis zone and back to the hydroxyl ammonium synthesis zone. Such equipment is schematically illustrated in Figure 1. The equipment as illustrated in Figure 1 comprises 3 parallel placed production units, however, the equipment may also comprise 2, 4, 5, 6 or even more parallel placed production units.

In each parallel placed hydroxyl ammonium production unit hydroxyl ammonium is produced by reduction of nitrate ions or nitrogen oxides with hydrogen in an inorganic process liquid in the presence of a catalyst. A suitable process is for instance disclosed in EP-A-1404613 and references cited therein. In general, the catalyst employed in the parallel placed hydroxyl ammonium production unit comprises a precious metal on a support, preferably platinum, palladium, or a combination of palladium and platinum on a support. Preferably, the support comprises carbon or alumina support, more preferably carbon. The catalyst employed in the parallel placed hydroxyl ammonium production unit preferably comprises between 1 to 25 wt.%, more preferably between 5 to 15 wt.% of the precious metal, relative to total weight of support plus catalyst. In general, the catalyst further comprises an activator. The activator is preferably selected from the group consisting of Cu, Ag, Au, Cd, Ga, In, TI, Ge, Sn, Pb, As, Sb and Bi, most preferably Ge. Generally, the activator is present in an amount of between 0.01 and 100 mg/g catalyst, preferably between 0.05 and 50 mg/g catalyst, more preferable between 0.1 and 10 mg/g catalyst, most preferably between 1 and 7 mg/g catalyst.

Generally, the catalyst is present in a hydroxyl ammonium production unit in an amount of 0.05-25 wt.%, preferable in an amount of 0.5-15 wt.%, more preferable in an amount of 0.2-5 wt.% relative to the total inorganic process liquid weight in the hydroxyl ammonium production unit. In general in the process according to the present invention an amount of catalyst is replaced while the total amount of catalyst is kept more or less constant. However, the process according to the present invention is not limited to maintaining the total amount of catalyst without increase or decrease. It is well possible to add catalyst in order to increase the total amount of catalyst or to add less catalyst after removal of a certain amount of catalyst. An increase or decrease in the total amount of catalyst can be used to further increase or decrease the production rate of hydroxyl ammonium.

Generally, the inorganic process liquid is an acidic, buffered inorganic process liquid comprising an aqueous reaction medium and a gaseous phase. Typically, the aqueous reaction medium is acidic, the pH preferably being between 0.5 and 6, more preferably between 1 and 4. Preferably, the aqueous reaction medium is buffered. Preferably, the aqueous reaction medium contains sulfuric acid or phosphoric acid, more preferably phosphoric acid. Preferably, the phosphate concentration in the aqueous reaction medium is higher than 2.0 mol/l and lower than 8.0 mol/l. More preferable higher than 2.5 mol/l and lower than 5 mol/l, most preferable higher than 3.5 mol/l and lower than 4.5 mol/l.

The nitrate (NO₃⁻) or nitrogen oxide may be reduced at any suitable temperature, for instance at a temperature ranging from 20 to 100 °C, preferably 30-90 °C, more preferably 40-65°C. Preferably, an aqueous product stream containing the hydroxyl ammonium formed is withdrawn from the aqueous product stream medium, the concentration hydroxyl ammonium in the aqueous product stream preferably ranges between 0.15 and 3.0 mol/l, more preferable between 0.5 and 2.5 mol/l, most preferable between 0.8 and 2.0 mol/l.

The inorganic process liquid leaving the hydroxyl ammonium production unit is filtered whereby the catalyst being separated off is returned into the hydroxyl ammonium production unit. Filtration can be performed by any known filtration method, e.g. by employment of a crossflow filtration technique as described in EP577213 or cake filtration as described in EP1257500.

Typically, the gaseous phase generally contains hydrogen, and non-hydrogen compounds, the composition being dependent on the relative flow rates of the hydrogen and non-hydrogen compounds to and from the reaction mixture and on the rate at which the reduction is effected. The relative volume of the gaseous phase may vary between wide ranges. Preferably, the volume percentage of the gaseous phase is between 15 to 50 vol.% (relative to the volume of aqueous reaction medium plus the volume of the gaseous phase plus the volume of the catalyst). The molar fraction of hydrogen in the gas mixture (with respect to the total molar quantity of all gaseous compounds in the gas mixture, i.e. the molar quantity of H₂ in the gas mixture divided by the sum molar quantity of all gaseous compounds in the gas mixture) is not limited to a specific value. A gas mixture may be withdrawn in which the molar fraction of hydrogen for example is higher than 0.35, preferably higher than 0.4, more preferably higher than 0.5, most preferably higher than 0.6. Increasing the molar fraction of hydrogen in the gas mixture has the advantage that the hydrogen partial pressure in the reaction mixture is brought to a higher level (for a constant total pressure). There is no specific upper limit for the molar fraction of hydrogen in the gas mixture. For practical reasons, the molar fraction of hydrogen in the gas mixture is usually lower than 0.95, in particular lower than 0.9.

In general the hydroxyl ammonium synthesis zone may be operated at atmospheric, sub-atmospheric or elevated pressures, preferably between 0.1 and 5 MPa, more preferably between 0.3 and 3 MPa, and in particular between 0.5 and 2 MPa (hydrogen partial pressure). An increased hydrogen partial pressure in the reaction mixture has the advantage that the activity and/or selectivity is increased. As used herein the hydrogen partial pressure in the reaction mixture refers to the molar fraction of hydrogen in the gas mixture multiplied by the total pressure in the reaction mixture. Preferably, the total pressure in the reaction mixture is higher than 1.5 MPa, more preferably higher than 2.0 MPa, most preferably higher than 2.5 MPa. The total pressure in the reaction mixture is preferably lower than 4.0 MPa, more preferably lower than 3.5 MPa.

The gas mixture withdrawn from the hydroxyl ammonium production unit comprises gaseous hydrogen (H₂) and gaseous non-hydrogen compounds. As used herein, hydrogen refers to H₂, and gaseous non-hydrogen compounds refer to gaseous compounds other than H₂. The gaseous non-hydrogen compounds may for example include CH₄, H₂O, NO, NO₂, N₂, and/or N₂O. The gaseous non-hydrogen compounds may for instance include by-products of the reduction (e.g. H₂O, NO, NO₂, N₂, and/or N₂O) and/or compounds which may be fed to the reaction zone together with the gaseous hydrogen (e.g. CH4, and/or N₂).

The molar fraction of non-hydrogen compounds in the gas mixture may vary between wide ranges. The molar fraction of N₂ in the gas mixture may for example be between 0.02 and 0.65, preferably between 0.05 and 0.5. If CH₄ is present in the gas mixture, the molar fraction of CH₄ in the gas mixture may for example be between 0 and 0.65, preferably between 0 and 0.5. The molar fraction of N₂O in the gas mixture may for example be between 0.001 and 0.08, preferably below 0.05, more preferably below 0.03. The molar fractions are given with respect to the sum molar quantity of all gaseous compounds in the gas mixture.

The production unit may be any suitable reactor, for instance a reactor with a mechanical stirrer or a column, most preferably a bubble column. An example of a suitable bubble column is described in NL-A-6908934.

In a preferred embodiment the inorganic process liquid comprising hydroxyl ammonium is fed to a cyclohexanone oxime synthesis zone. In such a cyclohexanone oxime synthesis zone hydroxyl ammonium is reacted with cyclohexanone in the presence of an organic solvent to form cyclohexanone oxime. Typically, the inorganic process liquid and cyclohexanone are fed to the cyclohexanone oxime synthesis zone, and an inorganic process liquid and the cyclohexanone oxime are withdrawn from the cyclohexanone oxime synthesis zone, whereby the inorganic process liquid is recycled to the hydroxyl ammonium synthesis zone.

A suitable process for the continuous production of cyclohexanone oxime is for instance described in EP 1303480, US 3997607 or in GB 1138750. The cyclohexanone oxime synthesis zone may be operated at a temperature ranging from 40 to 150°C and at atmospheric, sub-atmospheric, or elevated pressures, preferably between 0.05 and 0.5 MPa, more preferably between 0.1 and 0.2 MPa, most preferably between 0.1 and 0.15 MPa. Preferably, the aqueous reaction medium entering the cyclohexanone oxime synthesis zone has a pH of between 1 and 6, more preferably between 1.5 and 4.

Preferably, the inorganic process liquid leaving the cyclohexanone oxime synthesis zone is subjected to one or more separation steps prior to re-entering the hydroxyl ammonium synthesis zone in order to reduce the amount of organic contaminants, in particular cyclohexanone and cyclohexanone oxime. Preferably, the inorganic process liquid leaving the cyclohexanone oxime synthesis zone or leaving the extraction zone is subjected to stripping to achieve further reduction in organic contaminants. The stripping process described in US- 3940442 may for instance be used. It is preferred that the joint content of cyclohexanone and cyclohexanone oxime in the inorganic process liquid entering the hydroxyl ammonium synthesis zone is not more than 0.02 wt.% (200 ppm), more preferably not more than 0.005 wt.%, in particular not more than 0.002 wt.%, more in particular not more than 0.001 wt.% and most preferably not more than 0.0002 wt.% (relative to the weight of the inorganic process liquid).

### Brief Description of the Drawing

FIG. 1 is a schematic diagram of an embodiment of the process for the continuous production of cyclohexanone oxime according to the present invention.

### Description of an embodiment

Referring to FIG. 1, A represents the hydroxyl ammonium synthesis zone, whereas B represents a cyclohexanone oxime synthesis zone. Hydroxyl ammonium synthesis zone A comprises three parallel placed hydroxyl ammonium production units Aₐ, A_{b} and A_{c}. In zone A hydrogen is fed via line 1ₐ, 1_{b} and 1_{c} to production units Aₐ, A_{b} and A_{c}, respectively; unreacted hydrogen is discharged, with any other gases, from production units Aₐ, A_{b} and A_{c} via line 2ₐ, 2_{b} and 2_{c}, respectively. The inorganic process liquid, comprising phosphate and nitrate ions or nitrogen oxides, is fed to production units Aₐ, A_{b} and A_{c} in zone A through line 12_{a,} 12_{b} and 12_{c}, respectively. In production units Aₐ, A_{b} and A_{c} in zone A hydroxyl ammonium is produced in the inorganic process liquid. Subsequently, the inorganic process liquid comprising hydroxyl ammonium is passed through line 3ₐ, 3_{b} and 3_{c}, respectively, into line 4 and further into reaction zone B. In reaction zone B hydroxyl ammonium is reacted with cyclohexanone in order to produce cyclohexanone oxime. The cyclohexanone is fed in an organic solvent to the reaction zone B via line 5. The largest part of cyclohexanone oxime produced and dissolved in the organic solvent is removed from the system via line 6. The inorganic process liquid is passed to extraction zone C via line 7 in order to remove the last part of cyclohexanone oxime and organic solvent. The inorganic process liquid purified in extraction zone C is fed to zone D via line 8. Optional, the inorganic process liquid is fed via a stripping column into zone D. Such a stripping column is not illustrated in Figure 1. In zone D, nitric acid is produced. Preferably, nitric acid is produced, at zone D or thereafter, by reacting air, fed through line 9, with ammonia, fed through line 10, and water from the aqueous inorganic process liquid. Directly supplying nitric acid to the aqueous inorganic process liquid instead of producing nitric acid is also possible. Accordingly, the nitrate level is increased in the inorganic medium in zone D. The inorganic process liquid then completes the cycle by returning to hydroxyl ammonium synthesis zone A via line 11, where it is split into lines 12ₐ, 12_{b} and 12_{c}.

The following specific examples are to be construed as merely illustrative, and not limiting, of the remainder of the disclosure.

### Examples

### Comparative Experiment A

Through a 2 l glass reactor, an inorganic process liquid comprising a catalyst, 12g of a 10% Pd on carbon catalyst activated with 72 mg of germanium oxide per liter inorganic process liquid, is continuously recycled. The glass reactor is provided with a stirrer (1500 rpm), baffles, a gas inlet tube for hydrogen supply (150 l/h), a liquid supply, a condensor, a gas outlet and a liquid outlet provided with a filter system. The temperature in the glass reactor is 40 °C. The inorganic process liquid, having a pH value of 1.6, comprises 2.1 mol/kg H₃PO₄, 1.4 mol/kg NaNO₃ and 0.4 mol/kg HNO₃.

The hydroxyl ammonium production rate was measured by titrimetric measuring the hydroxyl ammonium concentration every 4 hours in the inorganic process liquid leaving the reactor. At the moment the initial hydroxyl ammonium production rate was decreased with 40%, the catalyst separated from 10% by weight of the content of the reactor was replaced by fresh catalyst 1.2g of a 10% Pd on carbon catalyst activated with 7.2 mg of germanium oxide. This replacement of catalyst was repeated every time the hydroxyl ammonium production rate was decreased to 40% of the initial level.

In this way an amount of hydroxyl ammonium was produced of 19.8 g/h, or recalculated on the amount of catalyst: 195 g/g catalyst.

### Comparative Experiment B

Comparative Experiment A was repeated whereby instead of 10% by weight of the content of the reactor 30% by weight was replaced by fresh catalyst (3.6g of a 10% Pd on carbon catalyst activated with 21.6 mg of germanium oxide). This replacement of catalyst was repeated every time the hydroxyl ammonium production rate was decreased to 40% of the initial level.

In this way an amount of hydroxyl ammonium was produced of 20.9 g/h, or recalculated on the amount of catalyst: 188 g/g catalyst.

### Example I

Through three 1 I glass reactors, each comprising 1/3 of the amount of inorganic process liquid recycled in comparative Experiment A, and thus comprising 4g of a 10% Pd on carbon catalyst activated with 24 mg of germanium oxide, inorganic process liquid is continuously recycles. The glass reactor is provided with a stirrer (1500 rpm), baffles, a gas inlet tube for hydrogen supply (150 l/h), a liquid supply, a condensor, a gas outlet and a liquid outlet provided with a filter system. The temperature in the glass reactor is 40°C. The inorganic process liquid, having a pH value of 1.6, comprises 2.1 mol/kg H₃PO₄, 1.4 mol/kg NaNO₃ and 0.4 mol/kg HNO₃.

The hydroxyl ammonium production rate was measured as described under comparative Experiment A. At the moment the initial hydroxyl ammonium production rate was decreased with 40%, in one glass reactor the catalyst was separated and replaced by fresh catalyst 4g of a 10% Pd on carbon catalyst. This replacement of catalyst was repeated every time the initial hydroxyl ammonium production rate was decreased with 40%, and each time in another reactor.

In this way an amount of hydroxyl ammonium was produced of 20.9 g/h, or recalculated on the amount of catalyst: 209 g/g catalyst. Compared to Comparative Experiment B this means that per g catalyst 9% more hydroxyl ammonium is produced.

### Example II

Example I was repeated with the only difference that instead of three 1 l glass reactors, two 1 l glass reactors were used, each comprising 1/2 of the amount of inorganic process liquid recycled in comparative Experiment A, and thus comprising 6g of a 10% Pd on carbon catalyst activated with 36 mg of germanium oxide.

In this way an amount of hydroxyl ammonium was produced of 21.5 g/h, or recalculated on the amount of catalyst: 197 g/g catalyst. Compared to Comparative Experiment A this means an increase in production rate of more than 11%.

## Claims

1. Process for the continuous production of hydroxyl ammonium by reduction of nitrate ions or nitrogen oxides with hydrogen in the presence of a catalyst **characterized in that** the hydroxyl ammonium is produced in 2 or more parallel placed hydroxyl ammonium production units.

2. Process according to claim 1 **characterized in that** in one or more parallel placed hydroxyl ammonium production units catalyst is replaced whereby the production of hydroxyl ammonium is continued in at least one parallel placed hydroxyl ammonium production unit.

3. Process according to any one of claims 1-2 **characterized in that** hydroxyl ammonium is produced in a phosphoric acid buffered inorganic process liquid.

4. Process according to any one of claims 1-3 **characterized in that** the catalyst used is a platinum, palladium, or a combination of palladium and platinum on a support.

5. Process according to claim 4 **characterized in that** the support is a carbon or alumina support.

6. Process according to claim 4 **characterized in that** the catalyst used is palladium on carbon, whereby the catalyst comprises 5 to 15 wt.% of palladium and between 0.01 and 1 wt.% of germanium, relative to total weight of support plus catalyst.

7. Process according to any one of claims 1-6 **characterized in that** two or three hydroxyl ammonium production units are placed in parallel.

8. Process for the production of cyclohexanone oxime in which an inorganic process liquid is cycled from a hydroxyl ammonium synthesis zone to a cyclohexanone oxime synthesis zone and back to the hydroxyl ammonium synthesis zone, in which hydroxyl ammonium synthesis zone hydroxyl ammonium is formed by reduction of nitrate ions or nitrogen oxides with hydrogen in the presence of a catalyst, and in which cyclohexanone oxime synthesis zone hydroxyl ammonium is reacted with cyclohexanone to form cyclohexanone oxime, **characterized in that** in the hydroxyl ammonium synthesis zone hydroxyl ammonium is produced in accordance with any one of claims 1- 7.

9. Equipment for the production of cyclohexanone oxime according to the process of claim 8 comprising a hydroxyl ammonium synthesis zone and cyclohexanone oxime synthesis zone **characterized in that** the hydroxyl ammonium synthesis zone comprises 2 or more parallel placed hydroxyl ammonium production units.

10. Equipment according to claim 9 **characterized in that** the hydroxyl ammonium synthesis zone comprises 3 or 4 parallel placed hydroxyl ammonium production units.

## Patentansprüche

1. Verfahren zur kontinuierlichen Herstellung von Hydroxylammonium durch Reduktion von Nitrationen oder Stickstoffoxiden mit Wasserstoff in Gegenwart eines Katalysators, **dadurch gekennzeichnet, daß** man das Hydroxylammonium in 2 oder mehr parallel angeordneten Hydroxylammonium-Produktionseinheiten herstellt.

2. Verfahren nach Anspruch 1, **dadurch gekennzeichnet, daß** man in einem oder mehreren parallel angeordneten Hydroxylammonium-Produktionseinheiten Katalysator ersetzt, wobei die Herstellung von Hydroxylammonium in mindestens einer parallel angeordneten Hydroxylammonium-Produktionseinheit fortgesetzt wird.

3. Verfahren nach einem der Ansprüche 1-2, **dadurch gekennzeichnet, daß** man Hydroxylammonium in einer phosphorsäuregepufferten anorganischen Prozeßflüssigkeit herstellt.

4. Verfahren nach einem der Ansprüche 1-3, **dadurch gekennzeichnet, daß** es sich bei dem verwendeten Katalysator um Platin, Palladium oder eine Kombination von Palladium und Platin auf einem Träger handelt.

5. Verfahren nach Anspruch 4, **dadurch gekennzeichnet, daß** es sich bei dem Träger um einen Kohle- oder Aluminiumoxidträger handelt.

6. Verfahren nach Anspruch 4, **dadurch gekennzeichnet, daß** es sich bei dem verwendeten Katalysator um Palladium auf Kohle handelt, wobei der Katalysator 5 bis 15 Gew.-% Palladium und zwischen 0,01 und 1 Gew.-% Germanium, bezogen auf das Gesamtgewicht von Träger und Katalysator, enthält.

7. Verfahren nach einem der Ansprüche 1-6, **dadurch gekennzeichnet, daß** man zwei oder drei Hydroxylammonium-Produktionseinheiten parallel anordnet.

8. Verfahren zur Herstellung von Cyclohexanonoxim, bei dem man eine anorganische Prozeßflüssigkeit von einer Hydroxylammonium-Synthesezone zu einer Cyclohexanonoxim-Synthesezone und wieder zurück zur Hydroxylammonium-Synthesezone im Kreis führt, wobei in der Hydroxylammonium-Synthesezone durch Reduktion von Nitrationen oder Stickstoffoxiden mit Wasserstoff in Gegenwart eines Katalysators Hydroxylammonium gebildet wird und in der Cyclohexanonoxim-Synthesezone Hydroxylammonium mit Cyclohexanon zu Cyclohexanonoxim umgesetzt wird, **dadurch gekennzeichnet, daß** in der Hydroxylammonium-Synthesezone Hydroxylammonium nach einem der Ansprüche 1-7 hergestellt wird.

9. Vorrichtung zur Herstellung von Cyclohexanonoxim nach dem Verfahren von Anspruch 8 mit einer Hydroxylammonium-Synthesezone und einer Cyclohexanonoxim-Synthesezone, **dadurch gekennzeichnet, daß** die Hydroxylammonium-Synthesezone 2 oder mehr parallel angeordnete Hydroxylammonium-Produktionseinheiten aufweist.

10. Vorrichtung nach Anspruch 9, **dadurch gekennzeichnet, daß** die Hydroxylammonium-Synthesezone 3 oder 4 parallel angeordnete Hydroxylammonium-Produktionseinheiten aufweist.

## Revendications

1. Procédé de production continue d'hydroxylammonium par réduction d'ions nitrate ou d'oxydes d'azote avec de l'hydrogène en présence d'un catalyseur, **caractérisé en ce que** l'hydroxylammonium est produit dans 2 ou plus de 2 unités de production d'hydroxylammonium placées en parallèle.

2. Procédé selon la revendication 1, **caractérisé en ce que**, dans une ou plusieurs des unités de production d'hydroxylammonium placées en parallèle, le catalyseur est remplacé, moyennant quoi la production d'hydroxylammonium est poursuivie dans au moins une des unités de production d'hydroxylammonium placées en parallèle.

3. Procédé selon l'une quelconque des revendications 1-2, **caractérisé en ce que** l'hydroxylammonium est produit dans un liquide de procédé inorganique tamponné à l'acide phosphorique.

4. Procédé selon l'une quelconque des revendications 1-3, **caractérisé en ce que** le catalyseur utilisé est du platine, du palladium, ou une combinaison de palladium et de platine, sur un support.

5. Procédé selon la revendication 4, **caractérisé en ce que** le support est un support de carbone ou d'alumine.

6. Procédé selon la revendication 4, **caractérisé en ce que** le catalyseur utilisé est du palladium sur carbone, moyennant quoi le catalyseur comprend 5 à 15% en poids de palladium et entre 0,01 et 1% en poids de germanium, par rapport au poids total du support plus catalyseur.

7. Procédé selon l'une quelconque des revendications 1-6, **caractérisé en ce que** deux ou trois unités de production d'hydroxylammonium sont placées en parallèle.

8. Procédé de production d'oxime de cyclohexanone dans lequel un liquide de procédé inorganique est mis à circuler depuis une zone de synthèse d'hydroxylammonium à une zone de synthèse d'oxime de cyclohexanone et renvoyé vers la zone de synthèse d'hydroxylammonium, dans laquelle zone de synthèse d'hydroxylammonium l'hydroxylammonium est formé par réduction d'ions nitrate ou d'oxydes d'azote avec de l'hydrogène en présence d'un catalyseur, et dans laquelle zone de synthèse d'oxime de cyclohexanone l'hydroxylammonium est mis à réagir avec de la cyclohexanone pour former l'oxime de cyclohexanone, **caractérisé en ce que**, dans la zone de synthèse d'hydroxylammonium, de l'hydroxylammonium est produit selon l'une quelconque des revendications 1-7.

9. Installation permettant de produire de l'oxime de cyclohexanone par le procédé de la revendication 8 comprenant une zone de synthèse d'hydroxylammonium et une zone de synthèse d'oxime de cyclohexanone, **caractérisée en ce que** la zone de synthèse d'hydroxylammonium comprend 2 ou plus de 2 unités de production d'hydroxylammonium placées en parallèle.

10. Installation selon la revendication 9, **caractérisée en ce que** la zone de synthèse d'hydroxylammonium comprend 3 ou 4 unités de production d'hydroxylammonium placées en parallèle.
